(19) Europäisches Patentamt European Patent Office Office européen des brevets

(11) **EP 2 812 037 B1**

(12) **EUROPÄISCHE PATENTSCHRIFT**

(45) Veröffentlichungstag und Bekanntmachung des Hinweises auf die Patenterteilung:
**09.08.2017   Patentblatt 2017/32**

(21) Anmeldenummer: **13706167.7**

(22) Anmeldetag: **23.01.2013**

(51) Int Cl.:
**A61L 24/00** (2006.01)      **A61L 24/08** (2006.01)
**A61L 24/10** (2006.01)      **D01D 10/00** (2006.01)
**A61L 26/00** (2006.01)

(86) Internationale Anmeldenummer:
**PCT/EP2013/000198**

(87) Internationale Veröffentlichungsnummer:
**WO 2013/117298 (15.08.2013 Gazette 2013/33)**

(54) **BIODEGRADIERBARES VLIES FÜR MEDIZINISCHE ZWECKE**

BIODEGRADABLE NON-WOVEN MATERIAL FOR MEDICAL PURPOSES

NON-TISSÉ BIODÉGRADABLE À USAGE MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2012   DE 102012002209**

(43) Veröffentlichungstag der Anmeldung:
**17.12.2014   Patentblatt 2014/51**

(73) Patentinhaber:
• **Heraeus Medical GmbH**
  **61273 Wehrheim (DE)**
• **Carl Freudenberg KG**
  **69465 Weinheim (DE)**

(72) Erfinder:
• **VOGT, Sebastian**
  **99092 Erfurt (DE)**

• **Hohmann, Ekaterini**
  **60598 Frankfurt am Main (DE)**
• **GRAFAHREND, Dirk**
  **68165 Mannheim (DE)**
• **REIBEL, Denis**
  **67850 Herrlisheim (FR)**
• **NEUMÜLLER, Daniel**
  **69469 Weinheim-Lützel-Sachsen (DE)**

(74) Vertreter: **Heraeus IP**
**Heraeus Holding GmbH**
**Schutzrechte**
**Heraeusstraße 12-14**
**63450 Hanau (DE)**

(56) Entgegenhaltungen:
**WO-A2-2009/126870      US-A1- 2008 076 722**
**US-A1- 2009 192 429**

EP 2 812 037 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein biodegradierbares Vlies, ein Verfahren zur Herstellung eines solchen biodegradierbaren Vlieses sowie die Verwendung eines solchen biodegradierbaren Vlieses als lokales Hämostyptikum.

In der Chirurgie kommt es häufig zu lokalen Blutungen im Weichgewebe, die nicht mit üblichen Methoden der Blutstillung, wie direktem Druck, Nähen, Klammern oder Kauterisierung gestoppt werden können. Durch eine effektive Hämostase können bei operativen Eingriffen die Anzahl der benötigten Transfusion deutlich reduziert werden sowie die Visualisierung des Eingriffsortes verbessert und die Operationszeit verkürzt werden. Zusätzlich lässt sich durch effektive Hämostase die Mortalität und Morbidität der Patienten während und nach operativen Eingriffen senken. Daher wurden Schwämme, Folien, Gazen und Pulver aus Kollagen, Cellulose und/oder Gelatine als lokale, passive Hämostyptika entwickelt.

Nachteilig ist speziell bei den Pulverformen, dass diese häufig aufgrund elektrostatischer Effekte an Handschuhen und Instrumenten der operierenden Ärzte haften bleiben und daher schwierig in der Handhabung sind. Die hämostyptische Wirkung von solchen Schwämmen, aber auch Folien und Gazen, beruht auf der Thrombozytenaggregation an der Oberfläche der Proteine oder der Cellulose, aus denen diese aufgebaut sind. Hierdurch werden ein Thrombus und ein effektiver Verschluss des Defekts ermöglicht. Im Falle der Kollagenhämostyptika ist zu berücksichtigen, dass zwischen 2% und 4% der Gesamtbevölkerung allergisch auf bovines Kollagen reagieren [A. K. Lynn, I. V. Yannas, W. Bonfield; Antigenicity and immunogenicity of collagen; J Biomed Mater Res B Appl Biomater. 2004; 71(2); 343-354]. Cellulose basierte Produkte enthalten regenerierte, oxidierte Cellulose. In der Literatur finden sich Hinweise, dass diese nicht so gut wie Kollagen und Gelatine-basierte Produkte resor- biert werden. Einige Fallstudien haben gezeigt, dass Rückstände der oxidierten Cellulose bei erneuter Operation (Revision) identifiziert werden konnten [Y. Tomizawa; Clinical benefits and risk analysis of topical hemostats: a review; J. Artif. Organs. 2005; 8(3); 137-142]. Die Eigenschaften von Gelatine erlauben die Anwendung auch bei irregulär geformten Wundgeometrien. Wenn ein solches Hämostyptikum am Blutungsort fixiert wird, passt es sich der Wunde an und quillt, wodurch in begrenzten Räumen ein Tamponadeeffekt auftritt. Gequollene Gelatine reduziert den Blutfluss und bildet eine stabile Matrix, um welche durch Kontaktaktivierung ein Thrombus gebildet wird. Im Wesentlichen existieren Gelatine-basierte Produkte bovinen und porcinen Ursprungs.

Nachteilig, bezogen auf die Handhabung während operativer Eingriffe, ist die hohe Klebrigkeit der blutgetränkten Produkte an chirurgischen Instrumenten [S. Srinath; Topical hemostatic agents in surgery: A surgeon's perspective; Aorn Journal. 2008; 88(3) 2-11]. Hergestellt werden die Schwämme im Allgemeinen durch Gefriertrocknung und auch durch spezielle Schäumungsprozesse. Nachteilig ist jedoch hieran, dass im Rahmen der Wundheilung Fibroblasten nur schwer oder auch nicht in die Schwämme und gequollene Pulver einwandern können. Allgemein wurden bei solchen rein passiven Hämostyptika auf der Basis von Kollagen, Gelatine und besonders im Falle der Cellulose Komplikationen bei Verwendung zu großer Mengen beobachtet. Produktrückstände können am Einsatzort Fremdkörperreaktionen, chronische Entzündungen und/oder Infektionen auslösen, welche wiederum Granulomabildung begünstigen und eine optimale Heilung verhindern. Granuloma wurden an verschiedensten Einsatzorten bei rein passiven Hämostyptika beobachtet [H. E. Achneck, B. Sileshi, R. M. Jamiolkowski, D. A. Albala, M. L. Shapiro, J. H. Lawson; A comprehensive review of topical hemostatic agents: Efficacy and recommendations for use; Anals of Surgery. 2010; 251(2). 217-228].

Die Blutgerinnung wird in die primäre Hämostase und in die sekundäre Hämostase unterteilt. Der wesentliche Schritt der primären Hämostase ist die Aggregation der Thrombozyten, die zu einem ersten Verschluss der Blutung führt. Die sekundäre Hämostase ist ein komplexer Kaskadenprozess an dessen Ende durch die Protease Thrombin und aus Fibrinogen Fibrin freigesetzt wird, das unter Quervernetzung ein stabiles Fibrinnetzwerk ausbildet. Die sekundäre Hämostase kann unter anderem durch Calcium-Ionen, den Faktor IV, ausgelöst werden.

Es wurde eine Reihe von aktiven Hämostyptika auf der Basis von Kollagenschwämmen vorgeschlagen, die Thrombin enthalten, um bei Kontakt mit Blut die Fibrin-Bildung aus Fibrinogen zu aktivieren. Solche aktive Hämostyptika zeigen biologische Aktivität und greifen direkt in die späteren Phasen des komplexen Kaskadenprozesses ein, um einen Thrombus an der Blutungsstelle zu induzieren. Dadurch soll eine rasche Stillung der Blutung erreicht werden. Eine effektive Blutstillung mit Thrombin setzt die Anwesenheit von Fibrinogen im Blut des Patienten voraus und versagt daher bei Patienten mit Afibrinogenemia. Kritisch ist jedoch zu sehen, dass insbesondere bei Verwendung von humanem Thrombin, dieses so behandelt sein muss, dass eine Übertragung von Viren, wie HIV und HCV, sicher ausgeschlossen werden kann. Zudem wurde das Potenzial bovinen und humanen Thrombins beobachtet, Antikörper zu induzieren (in bis zu 94% der Fälle) [H. Seyedejad, M. Imani, T. Jamieson, A. M. Seifalian; Topical haemostatic agents; British Journal of Surgery; 2008; 95; 1197-1225]. Obwohl viele Patienten keine klinischen Abnormalitäten nach der Entwicklung von Antikörpern zeigen, wurden Abnormalitäten in Blutkoagulationstests beobachtet, welche in Ausnahmefällen sogar fatale Folgen hatten (Anaphylaxis, Koagulopathie) [Y. Wai, V. Tsui, Z. Peng, R. Richardson, D. Oreopoulos, S. M. Tarlo; Anaphylaxis from topical bovine thrombin during haemodialysis and evaluation of sensitization among dialysis poulation; Clin Exp Allergy; 2003; 33; 1730-1734; M. Pope, K. W. Johnston; Anaphylaxis after thrombin injection of a femoral pseudoaneurysm: recommendations for prevention; J Vase Surg; 2000; 32; 190-191; und K. Tadokoro, T.Ohtoshi, S.

Takafuiji, K. Nakajima, S. Suzuki, K. Yamamoto et al.; Topical thrombin-induced IgE-mediated anaphylaxis: RAST analysis and skin-test studies; J Allergy Clin Immunol 1991; 88; 620-629]. US 2008/076722 A1 offenbart einen Verband mit Fasern aus Stärke, die ein Polymer zum induzieren der primären Hämostase ist. Der Verband enthält einen Aktivator der sekundären Hämostase, nämlich Calciumchlorid. Im Falle des bovinen Thrombins wurden zusätzlich bei An- wendungen im menschlichen Organismus starke Immunabwehrreaktionen beobachtet.

Im humanen Organismus gibt es als Antagonisten zu der sekundären Hämostase die Protease Plasmin. Plasmin spaltet Fibrinnetzwerke in kleine Bruchstücke. Dieser Prozess der Fibrinolyse wirkt der sekundären Hämostase entgegen.

Nachteilig an den bisher bekannten hämostyptischen Vliesen ist, dass die Blutungsstillung in manchen Fällen nicht schnell und effektiv genug erfolgt. Nachteilig an den hämostyptischen Vliesen nach dem Stand der Technik ist im Speziellen, dass die Wirkung der sekundären Hämostase durch die körpereigene Protease Plasmin reduziert wird und so die blutungsstillende Wirkung der hämostyptischen Vliese eingeschränkt ist.

Aufgabe der Erfindung ist es daher, ein verbessertes biodegradierbares Vlies zur Verfügung zu stellen, mit dem vorzugsweise die vorstehenden Nachteile überwunden werden. Insbesondere soll vorzugsweise ein biodegradierbares Vlies bereitgestellt werden, dass eine stärkere hämostyptische Wirkung als bisherige hämostyptische Vliese hat. Gleichzeitig soll das biodegradierbare Vlies einfach in der Anwendung und möglichst kostengünstig in der Herstellung sein.

Insbesondere soll ein Vlies entwickelt werden, das sowohl die primäre als auch sekundäre Hämostase aktiviert und bei dem das entstandene Fibrinnetzwerk zusätzlich stabilisiert wird. Weiterhin sollte das Vlies so beschaffen sein, dass humane Fibroblasten in das Vlies einwandern können, damit sich im Zuge der Wundheilung Bindegewebe ausbilden kann. Das Vlies sollte möglichst keine, aus humanem Blut isolierte Proteine enthalten, um von vornherein eine Übertragung von Infektionserregern, insbesondere von humanen Viren zu vermeiden.

Ein weiteres Ziel besteht darin, das Vlies vorzugsweise so zu gestalten, dass der pH-Wert des Vlieses im physiologischen, neutralen pH-Bereich stabilisiert wird, damit die Wundheilung nicht durch pH-Verschiebung beeinträchtigt werden kann.

Das Vlies sollte weiterhin mit Antiinfektiva modifiziert werden können, damit ein lokaler Schutz des Vlieses vor mikrobieller Besiedlung erreicht werden kann.

Diese Aufgaben werden durch Bereitstellung eines biodegradierbaren Vlieses nach Anspruch 1 gelöst.

Dementsprechend stellt die Erfindung ein biodegradierbares Vlies bereit, enthaltend (i) wenigstens ein Polymer zum Induzieren der primären Hämostase, (ii) wenigstens einen nicht-proteinogenen, niedermolekularen, in Wasser löslichen Aktivator der sekundären Hämostase und (iii) wenigstens einen nicht-proteinogenen, niedermolekularen, in Wasser löslichen Inhibitor der Fibrinolyse.

Die Erfindung stellt ferner ein Verfahren zur Herstellung eines solchen biodegradierbaren Vlieses zur Verfügung, wobei (i) ein fluidisiertes Faserrohmaterial gegebenenfalls mit Additiven in ein Behältnis gegeben wird, (ii) das Behältnis in Rotation versetzt wird, (iii) das fluidisierte Faserrohmaterial durch Zentrifugalkräfte aus dem Behältnis ausgetragen wird, wodurch Fasern (1) oder Filamente (1) gebildet werden, und (iv) aus den Fasern (1) oder Filamenten (i) ein biodegradierbares Vlies gebildet wird.

Darüber hinaus stellt die Erfindung die Verwendung eines solchen biodegradierbaren Vlieses als lokales Hämostyptikum bereit.

Unter einem biodegradierbaren Material werden vorliegend Materialien, insbesondere Polymere, und Bauteile verstanden, die degradieren und in-vivo resorbiert werden. Die Materialien werden dabei über den natürlichen Stoffwechselweg eliminiert. Dies kann durch einfache Filtrationsvorgänge der Degradationsprodukte oder nach ihrer Metabolisierung erfolgen.

Das biodegradierbare Vlies enthält (i) ein Polymer zum Induzieren der primären Hämostase.

Das Polymer zum Induzieren der primären Hämostase ist vorzugsweise aus der Gruppe ausgewählt, die aus Kollagen, Gelatine, Carboxymethylcellulose, Oxycellulose, Carboxymethyldextran und Mischungen davon besteht. Diese Polymere sind leicht erhältlich und eignen sich besonders gut zum Aufbau des hämostyptischen Vlieses beziehungsweise der Fasern für das hämostyptische Vlies. Das Vlies enthält wenigstens einen nicht-proteinogenen, niedermolekularen, in Wasser löslichen Aktivator der sekundären Hämostase.

Der Aktivator der Hämostase ist nicht-proteinogen, wenn er keine $\alpha$-Aminosäuren, keine Peptide und keine Oligopeptide, vorzugsweise überhaupt keine Peptide umfasst.

Der Aktivator ist niedermolekular, wenn er eine molare Masse von weniger als 1000 g/mol aufweist. Vorzugsweise weist der Aktivator eine molare Masse von weniger als 800 g/mol, mehr bevorzugt von weniger als 500 g/mol und besonders bevorzugt von weniger als 200 g/mol auf.

Der Aktivator der sekundären Hämostase ist vorzugsweise in Wasser löslich, wenn die Löslichkeit des Aktivators der sekundären Hämostase in Wasser bei einer Temperatur von 25°C wenigstens 100 mg pro Liter, mehr bevorzugt wenigstens 500 mg pro Liter, noch mehr bevorzugt wenigstens 1000 mg pro Liter und besonders bevorzugt wenigstens 2000 mg pro Liter beträgt.

Dieser Aktivator der sekundären Hämostase wirkt vorzugsweise blutstillend, das heißt, er ist dazu geeignet, einer Blutung eines Patienten medizinisch entgegenzuwirken. Vorzugsweise unterstützt der Aktivator der sekundären Hämostase die körpereigene Hämostase, so dass es zu einer schnelleren Stillung der Blutung kommt.

Gemäß einer bevorzugten Ausführungsform ist der Aktivator der sekundären Hämostase wenigstens ein Calciumsalz. Dieses wenigstens eine Calciumsalz weist vorzugsweise eine Löslichkeit von mehr als 2 g/Liter in Wasser bei einer Temperatur von 25°C auf. Vorzugsweise ist das wenigstens eine Calciumsalz aus der Gruppe ausgewählt, die aus Calciumchlorid, Calciumacetat, Calciumsulfat-Dihydrat, Calciumlactat und Mischungen davon besteht. Calciumsalze sind besonders einfach beim Aufbau eines erfindungsgemäßen Vlieses einsetzbar. Zudem sind sie vom Organismus des Patienten leicht umsetzbar.

Der Anteil des Aktivators der sekundären Hämostase liegt vorzugsweise im Bereich von 0,1 bis 20 Gewichtsprozent, mehr bevorzugt im Bereich von 0,5 bis 15 Gewichtsprozent und noch mehr bevorzugt im Bereich von 1 bis 10 Gewichtsprozent, bezogen auf das Gewicht des Vlieses.

Das Vlies enthält erfindungsgemäß einen nicht-proteinogenen, niedermolekularen, in Wasser löslichen Inhibitor der Fibrinolyse.

Der Inhibitor der Fibrinolyse ist nicht-proteinogen, wenn er keine $\alpha$-Aminosäuren, keine Peptide und keine Oligopeptide, vorzugsweise überhaupt keine Peptide, umfasst.

Der Inhibitor der Fibrinolyse ist niedermolekular, wenn er eine molare Masse von weniger als 1000 g/mol aufweist. Vorzugsweise weist der Inhibitor der Fibrinolyse eine molare Masse von weniger als 800 g/mol, mehr bevorzugt von weniger als 500 g/mol und besonders bevorzugt von weniger als 200 g/mol auf.

Der Inhibitor der Fibrinolyse ist vorzugsweise in Wasser löslich, wenn die Löslichkeit des Inhibitors der Fibrinolyse in Wasser bei einer Temperatur von 25°C vorzugsweise wenigstens 100 mg pro Liter, mehr bevorzugt wenigstens 500 mg pro Liter, noch mehr bevorzugt wenigstens 1000 mg pro Liter und besonders bevorzugt wenigstens 2000 mg pro Liter beträgt.

Gemäß einer bevorzugten Ausführungsform der Erfindung ist der nicht-proteinogene, niedermolekulare, in Wasser lösliche Inhibitor der Fibrinolyse ein Analogon des Lysins. Vorzugsweise handelt es sich bei dem nicht-proteinogenen, niedermolekularen, in Wasser löslichen Inhibitor der Fibrinolyse um eine amphotere Aminocarbonsäure. Vorzugsweise ist der nicht-proteinogene, niedermolekulare, in Wasser lösliche Inhibitor der Fibrinolyse eine $\alpha$-Aminocarbonsäuren. Gemäß einer bevorzugten Ausführungsform befinden sich zwischen der Aminogruppe und der Carboxylgruppe der $\alpha$-Aminocarbonsäure wenigstens fünf Kohlenstoffatome und mehr bevorzugt genau fünf Kohlenstoffatome. Der wenigstens eine nicht-proteinogene, niedermolekulare, in Wasser lösliche Inhibitor der Fibrinolyse ist vorzugsweise aus der Gruppe ausgewählt, die aus 6-Aminohexansäure, 4-Aminomethylbenzoesäure, trans-4-Aminomethylcyclohexylcarbonsäure und Mischungen davon besteht. Diese Stoffe haben sich als besonders geeignet zur Herstellung des hämostyptischen Vlieses beziehungsweise zur Herstellung der Fasern für das hämostyptische Vlies herausgestellt. Gleichzeitig sind diese Stoffe aus medizinischer Sicht unbedenklich für den Patienten und damit in Medizinprodukten einsetzbar.

Es kann erfindungsgemäß auch vorgesehen sein, dass die Menge des nicht-proteinogenen, niedermolekularen, in Wasser löslichen Inhibitors der Fibrinolyse derart gewählt ist, dass dieser eine den pH-Wert stabilisierende Pufferwirkung im Bereich der Oberfläche des biodegradierbaren Vlieses hat, wobei vorzugsweise der Inhibitor den pH-Wert im Bereich zwischen 6 und 8 puffert.

Die Pufferwirkung durch den Inhibitor der Fibrinolyse ist besonders vorteilhaft, weil dann kein zusätzlicher Stoff als Puffer in das biodegradierbare Vlies eingebracht werden muss.

Vorzugsweise liegt der Anteil des Inhibitors der Fibrinolyse im Bereich von 0,1 bis 20 Gewichtsprozent, mehr bevorzugt im Bereich von 0,5 bis 15 Gewichtsprozent und noch mehr bevorzugt im Bereich von 1 bis 10 Gewichtsprozent, bezogen auf das Gewicht des Vlieses. Bei diesen Anteilen des Inhibitors der Fibrinolyse wird sowohl die Fibrinolyse ausreichend gehemmt, als auch der pH-Wert in einem für die Hämostase bevorzugten Bereich gehalten.

Gemäß einer bevorzugten Ausführungsform umfassen die Fasern des biodegradierbaren Vlieses (i) das Polymer zum Induzieren der primären Hämostase, (ii) den nicht-proteinogenen, niedermolekularen, in Wasser löslichen Aktivator der sekundären Hämostase und/oder (iii) den nicht-proteinogenen, niedermolekularen, in Wasser löslichen Inhibitor der Fibrinolyse. Vorzugsweise sind dabei (i) das Polymer zum Induzieren der primären Hämostase, (ii) der nicht-proteinogene, niedermolekulare, in Wasser lösliche Aktivator der sekundären Hämostase und/oder (iii) der nicht-proteinogene, niedermolekulare, in Wasser lösliche Inhibitor der Fibrinolyse homogen in en Fasern des Vlieses verteilt.

Die Fasern lassen sich besonders bevorzugt bereits mit dem Aktivator der sekundären Hämostase und dem Inhibitor der Fibrinolyse herstellen. Hierdurch werden Kosten beim Aufbau des Vlieses eingespart und es wird ein einfach aufgebautes, homogenes, hämostyptisches Vlies erhalten.

Ferner kann vorgesehen sein, dass das hämostyptische Vlies wenigstens einen antiinfektiven Wirkstoff aufweist.

Dieser wenigstens eine antiinfektive Wirkstoff ist vorzugsweise wenigstens ein Antibiotikum.

Der wenigstens eine antiinfektive Wirkstoff ist vorzugsweise in den Fasern des biodegradierbaren Vlieses enthalten. Der wenigstens eine antiinfektive Wirkstoff kann dabei auch auf der Vliesoberfläche angeordnet sein.

Der wenigstens eine antiinfektive Wirkstoff ist vorzugsweise in Wasser löslich. In Wasser löslich ist der wenigstens eine antiinfektive Wirkstoff vorzugsweise dann, wenn die Löslichkeit des wenigstens einen antiinfektiven Wirkstoffs in Wasser bei einer Temperatur von 25°C vorzugsweise wenigstens 100 mg pro Liter, mehr bevorzugt wenigstens 500 mg pro Liter, noch mehr bevorzugt wenigstens 1000 mg pro Liter und besonders bevorzugt wenigstens 2000 mg pro Liter beträgt. Vorzugsweise liegt der wenigstens eine antiinfektive Wirkstoff im Vlies in einer pharmazeutisch wirksamen Menge vor.

Es ist von besonderem Vorteil, wenn der wenigstens eine antiinfektive Wirkstoff bei der Herstellung des biodegradierbaren Vlieses mit in das Vlies eingebaut wird. Beispielsweise kann der wenigstens eine antiinfektive Wirkstoff dabei in das Fasermaterial zur Herstellung des biodegradierbaren Vlieses aufgenommen werden. Solche Vliese wirken dann zusätzlich entzündungshemmend und einer Infektion des Patienten entgegen.

Es kann erfindungsgemäß auch vorgesehen sein, dass das biodegradierbare Vlies eine in Wasser gering lösliche Puffersubstanz umfasst. Diese Puffersubstanz kann beispielsweise in den Fasern des Vlieses enthalten und dort gegebenenfalls homogen verteilt sein.

Vorzugsweise ist eine Puffersubstanz in Wasser gering löslich, wenn die Löslichkeit der Puffersubstanz in Wasser bei einer Temperatur von 25°C weniger als 10 g und mehr bevorzugt weniger als 5 g beträgt. Die Löslichkeit der Puffersubstanz in Wasser bei einer Temperatur von 25°C liegt vorzugsweise im Bereich von 1 mg/Liter bis 1 g/Liter und mehr bevorzugt im Bereich von 5 mg/Liter bis 500 mg/Liter.

Gemäß einer bevorzugten Ausführungsform ist die Puffersubstanz aus der Gruppe ausgewählt, die aus Calciumcarbonat, Magnesiumcarbonat, basischem Magnesiumcarbonat und Mischungen davon besteht.

Eine zusätzliche Puffersubstanz hilft bei der Einstellung eines für die Hämostase geeigneten pH-Werts im Blut des Patienten in der unmittelbaren Umgebung des in der Wunde eingesetzten biodegradierbaren Vlieses.

Eine weitere Ausgestaltung der Erfin- dung kann vorsehen, dass das biodegradierbare Vlies einen pH-Indikator umfasst. Dabei können insbesondere die Fasern des Vlieses einen pH-Indikator umfassen.

Dieser pH-Indikator weist vorzugsweise einen Umschlagpunkt bei einem pH-Wert von weniger als pH 7,4 auf. Vorzugsweise handelt es sich bei dem pH-Indikator um Bromkresolpurpur oder Bromthymolblau.

Mit Hilfe des pH-Indikators kann optisch überprüft werden, wie die Situation im Wundbereich ist oder ob weitere Behandlungsmaßnahmen erfolgen sollten, um eine schnelle Blutstillung zu erreichen.

Gemäß einer besonders bevorzugten Ausgestaltung der Erfindung beträgt die durchschnittliche Maschenweite zwischen den Fasern des trockenen Vlieses wenigstens 50 $\mu$m. Die durchschnittliche Maschenweite zwischen den Fasern des trockenen Vlieses liegt vorzugsweise im Bereich von 50 $\mu$m bis 500 $\mu$m und mehr bevorzugt im Bereich von 100 $\mu$m bis 200 $\mu$m.

Durch eine Maschenweite in diesem Bereich wird das Einwachsen von Fibroblasten erleichtert. Dies bewirkt eine schnellere Wundheilung bei der Verwendung eines erfindungsgemäßen biodegradierbaren Vlieses.

Es kann erfindungsgemäß auch vorgesehen sein, dass die Fasern des biodegradierbaren Vlieses einen mittleren Faserdurchmesser im Bereich von 0,5 $\mu$m bis 500 $\mu$m vorzugsweise im Bereich von 2 $\mu$m bis 300 $\mu$m und mehr bevorzugt im Bereich von 5 $\mu$m bis 200 $\mu$m aufweisen.

Diese Faserdurchmesser sind ausreichend stark, um ein Abbrechen einzelner Fasern zu verhindern und um eine ausreichende Oberfläche des biodegradierbaren Vlieses zur Verfügung zu stellen, um die Wirkstoffe in ausreichenden Konzentrationen abzugeben.

Das erfindungsgemäße biodegradierba- re Vlies kann beispielsweise als lokales Hämostyptikum verwendet werden.

Der Erfindung liegt in Teilen die überraschende Erkenntnis zugrunde, dass durch die Kombination eines Aktivators der sekundären Hämostase mit einem Inhibitor der Fibrinolyse als wasserlösliche Bestandteile eines biodegradierbaren Vlieses gelingt, die hämostyptische Wirkung zu verbessern.

Wie bereits erwähnt, gibt es im humanen Organismus als Antagonisten zu der sekundären Hämostase die Protease Plasmin, die das Fibrinnetzwerke in kleine Bruchstücke aufspaltet.

Der Prozess der Fibrinolyse wirkt so der sekundären Hämostase entgegen. Die Bildung von Plasmin wird durch Plasminogenaktivatoren induziert.

Das Plasmin kann durch Analoga der Aminosäure L-Lysin gehemmt werden (K. Aktories, U. Förstermann, W. Forth; Allgemeine und spezielle Pharmakologie und Toxikologie; Elsevier, Urban&Fischer Verlag, 9. Auflage, 2006; 547). Bekannt sind dazu unter anderem $\alpha$-Aminocarbonsäuren, wobei sich zwischen der Aminogruppe und der Carboxylgruppe fünf Kohlenstoffatome befinden müssen. Auf dem Markt befindliche hämostyptische Präparate mit dem antifibrinolytischen Stoff Aprotinin weisen wesentliche Nachteile auf. Bovines Aprotinin kann anaphylaktische Reaktionen auslösen [R. N. Kaddoum, E. J. Chidiac, M. M. Zestos, S. D. Rajan, A. Baraka; An anaphylactic reaction after primary exposure to an aprotinin test dose in a child with a severe milk allergy; J. Cardiothorac. Vasc. Anesth.; 2007; 21; 243-244]. Zwischen 0,9% und 2,6% der Patienten, die mit bovinem Aprotinin behandelt wurden, zeigten bei wiederholter Exposition Hypersensitivierungsreaktionen [W. Dietrich, A. Ebell, R. Busley, A. Boulesteix; Aprotinin and anaphylaxis: analysis of 12403 exposures to aprotinin in cardiac surgery; Ann. Thorac. Surg.; 2007; 84; 1144-1150].

Es wurde nun im Rahmen der Erfin- dung überraschend gefunden, dass Inhibitoren der Fibrinolyse, wie beispielsweise Analoga des Lysins, als wasserlösliche (und damit im Blut lösliche) Bestandteile hämostyptischer Vliese verwendet werden können, um die hämostyptische Wirkung des Vlieses zu verbessern.

Die Erfindung beruht ferner in Teilen auf dem überraschenden Effekt, dass Lysin-Analoga, als niedermolekulare Inhibitoren der Fibrinolyse, bei Inkorporation entsprechender Mengen in das Vlies, den pH-Wert der Oberfläche und der unmittelbaren Umgebung des Vlieses auf einen physiologischen, annähernd neutralen pH-Bereich puffern.

Es gibt im Stand der Technik kein lokal hämostyptisches Material in Form von Schwämmen, Geweben oder Vliesen, welches neben der Induktion der primären und sekundären Hämostase gleichzeitig die Fibrinolyse des gebildeten Fibrin-Netzwerks hemmt.

Durch die Erfindung wird ein biodegradierbares Vlies bereitgestellt, das sowohl die primäre als auch sekundäre Hämostase aktiviert und bei dem das entstandene Fibrinnetzwerk zusätzlich durch Inhibierung der Fibrinolyse stabilisiert wird.

Auch existiert im Stand der Technik kein lokal hämostyptisches Material, das den pH-Wert in der unmittelbaren Umgebung des hämostyptischen Materials gezielt durch einen Puffer-Zusatz im Neutralbereich einstellt und so die Hämostase auch durch diese Maßnahme fördert.

Der pH-Wert des Wundsekrets beeinflusst den Prozess der Wundheilung. Für eine optimale Wundheilung ist ein neutraler pH-Wert vorteilhaft (J. Dissemond; Die Bedeutung des pH-Wertes für die Wundheilung; HARTMANN wundForum 1/2006; 15-19). Daher ist die Einstellung des pH-Werts innerhalb eines gewünschten Bereichs erfindungsgemäß auch bei Einsatz erfindungsgemäßer Vliese vorteilhaft. Hierzu wird eine lösliche Puffersubstanz auf das Vlies aufgetragen. Besonders vorteilhaft ist es und ein besonderer kombinatorischer Effekt wird erzielt, wenn als Puffersubstanz der ohnehin verwendete niedermolekulare Inhibitor der Fibrinolyse verwendet wird. Hierzu muss lediglich eine ausreichende Menge des Inhibitors auf die Oberfläche des Vlieses aufgebracht werden, so dass dieser eine den pH-Wert stabilisierende Pufferwirkung im unmittelbaren Bereich der Oberfläche des Vlieses bewirkt.

Das erfindungsgemäße biodegradierbare Vlies kann vorzugsweise durch das hierin beschriebene Verfahren zur Herstellung eines biodegradierbaren Vlieses hergestellt werden.

Bei diesem Verfahren wird (i) ein fluidisiertes Faserrohmaterial gegebenenfalls mit Additiven in ein Behältnis gegeben, (ii) das Behältnis in Rotation versetzt, (iii) das fluidisierte Faserrohmaterial durch Zentrifugalkräfte aus dem Behältnis ausgetragen, wodurch Fasern (1) oder Filamente (1) gebildet werden, und (iv) aus den Fasern (1) oder Filamenten (i) ein biodegradierbares Vlies gebildet. Bei dem Behältnis, in das das fluidisierte Faserrohmaterial gegebenenfalls mit Additiven eingebracht wird, handelt es sich vorzugsweise um einen Spinnrotor.

Dieses Herstellungsverfahren ist besonders einfach und kostengünstig zu realisieren.

Dabei kann vorgesehen sein, dass die erzeugten Fasern oder Filamente aus dem rotierenden Behältnis als ein Flächenmaterial auffangen werden, in dem sich in einer Vielzahl von Bereichen des Flächenmaterials Verbindungsstellen zwischen zwei oder mehreren Fasern bilden.

Auch diese Maßnahme dient dazu, das Herstellungsverfahren einfach und kostengünstig zu halten.

Ferner kann vorgesehen sein, dass das Vlies, insbesondere das Flächenmaterial in wenigstens einem Nachbehandlungsschritt mit wenigstens einem flüssigen Medium getränkt und/oder beschichtet wird, wobei insbesondere als flüssiges Medium biologisch abbaubare Polymermateria- lien und/oder wachsartige Materialien verwendet werden.

Erfindungsgemäße biodegradierbare Vliese können mit einem Rotationsspinnverfahren, zum Beispiel nach der DE 10 2007 011 606 A1; der WO 2011/116848 und der DE 10 2007 044 648 A1, einfach und kostengünstig hergestellt werden.

Die von dem Spinnrotor erzeugten Fasern beziehungsweise Filamente lassen sich einfach in einem Zustand auffangen, indem sich in einer Vielzahl von Bereichen des Flächenmaterials Verbindungsstellen zwischen zwei oder mehreren Fasern bilden.

In einem optionalen Nachbehandlungsschritt lässt sich das erfindungsgemäße Flächenmaterial in einer Vielzahl von Eigenschaften an spezifische Applikationen anpassen.

Durch Vernetzung des Materials können die mechanischen und insbesondere chemischen Eigenschaften des biodegradierbaren Vlieses modifiziert werden. Beispielsweise lassen sich über den Vernetzungsgrad des Materials die Resorptionseigenschaften für medizinische Anwendungszwecke vorgeben.

Die erfindungsgemäßen Flächenmaterialien lassen sich in Nachbehandlungsschritten mit flüssigen Medien tränken und/oder beschichten. Dafür kommen insbesondere, aber nicht ausschließlich andere biologisch abbaubare Polymermaterialien oder auch wachsartige Materialien in Frage.

Mittels des vorstehend beschriebenen erfindungsgemäßen Verfahrens lassen sich auf einfache Weise Faser-Flächenmaterialien für erfindungsgemäße biodegradierbare Vliese erzeugen, die Fasern mit einer mittleren Faserdicke von 0,5 $\mu$m bis 500 $\mu$m haben.

Zur Erzeugung von partiell vernetzten Materialien in den Fasern wird bevorzugt bereits der Spinnlösung ein Vernetzungsmittel zugefügt. Eine Vernetzung lässt sich allerdings auch und zusätzlich bei den bereits versponnenen Fasern durch in Kontakt bringen mit einem Vernetzungsmittel, sei es gasförmig oder in Lösung, bewirken.

Am bereits fertig vorliegenden Wirrgelege kann erfindungsgemäß eine weitere Vernetzung vorgenommen werden, die dann den endgültigen Vernetzungsgrad der Fasern in dem Flächenmaterial bestimmt und damit dessen biologische Abbaurate.

Für die Vernetzung stehen verschiedene Verfahren zur Verfügung, wobei enzymatische Verfahren, die Verwendung von Komplexbildnern oder chemische Verfahren bevorzugt sind.

Bei der chemischen Vernetzung wird die Vernetzung mittels einem oder mehreren Reaktanden durchgerührt, insbesondere mit Aldehyden, ausgewählt aus Formaldehyd und Dialdehyden, Isocyanaten, Diisocyanaten, Carbodiimiden, Alkyldihalogeniden. Ferner können hydrophile Di- und Trioxirane wie zum Beispiel 1,4-Butandioldiglycidether, Glycerintriglycidether und Polyethylenglycolderivate eingesetzt werden. Besonders bevorzugt ist hierbei die Verwendung von Polyethylenglycoldiglycidylether. Neben der Vernetzung zeigten Polyethylenglycolderivate die positive Eigenschaft, ungewollte Adhäsionen zu vermeiden, zum Beispiel Pericardadhäsionen im Falle von Herzoperationen [W. F. Konertz, M. Kostelka, F. W. Mohr et al.; Reducing the incidence and severity of pericardial adhesions with a sprayable polymeric matrix; Ann. Thorac. Surg.; 2003; 76; 1270-1274].

Insbesondere bei der medizinischen Anwendung empfiehlt es sich, nach dem Vernetzen überschüssiges Vernetzungsmittel aus dem Flächenmaterial beziehungsweise dem Wirrgelege zu entfernen.

Wie zuvor beschrieben, ist es bevor- zugt, der Spinnlösung bereits ein Vernetzungsmittel zuzugeben und dann an dem fertigen Flächenmaterial, sozusagen in einer zweiten Stufe, eine weitere Vernetzung bis zum gewünschten Vernetzungsgrad durchzuführen.

Es kann erfindungsgemäß vorgesehen sein, dass die Porosität s eines biodegradierbaren Vlieses durch die folgende Formel gegeben ist oder berechnet wird:

$$\varepsilon = 1 - \frac{\rho_{Vlies}}{\rho_{Bulk}}$$

$$= 1 - \frac{\frac{m_{Vlies}}{V_{Vlies}}}{\rho_a \cdot \frac{m_a}{(m_a + m_b + m_c)} + \rho_b \cdot \frac{m_b}{(m_a + m_b + m_c)} + \rho_c \cdot \frac{m_c}{(m_a + m_b + m_c)}}$$

Darin ist $\rho_{Vlies}$ die Dichte des nicht komprimierten biodegradierbaren Vlieses, $\rho_{Bulk}$ die Dichte der Fasern des biodegradierbaren Vlieses, $m_{Vlies}$ die Masse des biodegradierbaren Vlieses, $V_{Vlies}$ das Volumen des biodegradierbaren Vlieses, $\rho_a$ die Dichte des faserbildenden Polymers, $\rho_b$ die Dichte des Aktivators der sekundären Hämostase, $\rho_c$ die Dichte des Inhibitors der Fibrinolyse, $m_a$ die Masse des faserbildenden Polymers im Vlies, $m_b$ die Masse des Aktivators der sekundären Hämostase im Vlies und $m_c$ die Masse des Inhibitors der Fibrinolyse im Vlies.

Wenn noch weitere Komponenten, wie beispielsweise eine zusätzliche Puffersubstanz oder Antibiotika im biodegradierbaren Vlies enthalten sind und die Porosität des biodegradierbaren Vlieses bestimmt werden soll, müssen noch weitere Parameter nach obigem Muster berücksichtigt werden, das heißt die Massen ($m_d$, $m_e$, ...) und Dichten ($\rho_d$, $\rho_e$, ...) der zusätzlichen Komponenten.

Erfindungsgemäß ist besonders bevorzugt, dass die Faseroberfläche $O_{Faser}$ nach folgender Formel berechnet wird oder gegeben ist durch:

$$O_{Faser} = \pi \cdot \emptyset_{Faser} \cdot \frac{1 - \varepsilon \cdot V_{Vlies}}{\pi \left(\frac{\emptyset_{Faser}}{2}\right)^2},$$

wobei $\emptyset_{Faser}$ der durchschnittliche Durchmesser der Fasern ist.

Im Folgenden werden Ausführungsbeispiele der Erfindung anhand von vier schematisch dargestellten Figuren erläutert, ohne jedoch dabei die Erfindung zu beschränken. In den Ausführungsbeispielen werden die Begriffe Vlies und Vliesstoff synonym verwendet. Dabei zeigt:

Figur 1:     einen erfindungsgemäßen Gelatinevliesstoff mit trockenem, antimikrobiellem Stoff;
Figur 2:     einen erfindungsgemäßen Gelatinevliesstoff mit nassem, antimikrobiellem Stoff in Wasser;
Figur 3:     einen erfindungsgemäßen Gelatinevliesstoff mit antimikrobieller Beschichtung; und
Figur 4:     einen erfindungsgemäßen Gelatinevliesstoff mit antimikrobiellem Stoff und antimikrobieller Beschichtung.

AUSFÜHRUNGSBEISPIEL 1:

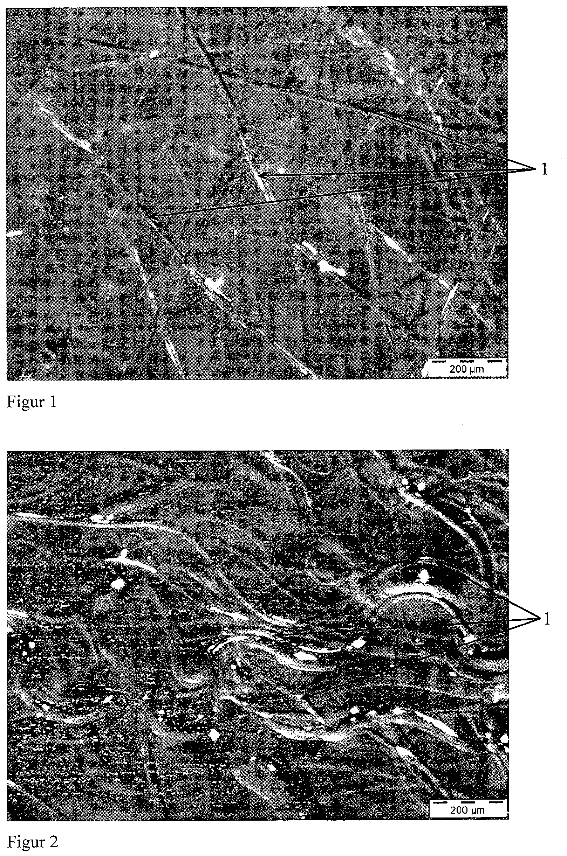

Die Figuren 1 und 2 zeigen als erstes Ausführungsbeispiel einen erfindungsgemäßen Gelatinevliesstoff mit trockenem, antimikrobiellem Stoff (Figur 1) und den erfindungsgemäßen Gelatinevliesstoff mit nassem, antimikrobiellem Stoff (Figur 2). Der Vliesstoff umfasst Fasern 1 oder Filamente 1, die als Wirrgelege übereinander liegen miteinander vernetzt sind. Im nassen Zustand nach Figur 2 ist zu erkennen, dass die nassen Fasern 1 aufgrund der Einwirkung der Flüssigkeit stärker gekrümmt sind, als im trockenen Zustand.

Der Gelatinevliesstoff mit antimikrobiellem Stoff gemäß Figur 1 (trocken) und Figur 2 (nass; nach sechs Stunden in destilliertem Wasser) wird durch ein Rotationsspinnverfahren wie folgt hergestellt:

Zunächst wird eine 24%ige Gelatine-Lösung hergestellt. Zur Verwendung kommt beispielsweise eine Gelatine des Typs A PIGSKIN der Firma GELITA AG in Frage, die für das vorliegende Ausfüh- rungsbeispiel verwendet wurde. Die Gelatine wird in Wasser eingerührt. Der pH-Wert wird mit NaOH (Produktnummer: 3306576, Sigma-Aldrich, Germany) auf 7,4 eingestellt. Der Gelatine-Lösung werden 1 Gew% Calciumchlorid (Produktnummer: 102382, Merck, Germany), 1 Gew% Calciumcarbonat (Produktnummer: C4830, Sigma-Aldrich, Germany), 1 Gew% Glycerin (Produktnummer: 01873, Sigma-Aldrich, Germany) und 0,5 Gew% 6-Aminohexansäure (Produktnummer: 800145, Merck, Germany) zugegeben.

Darauf verbleibt die Gelatine-Lösung etwa eine Stunde in Ruhe, um aufzuquellen. Anschließend wird die Gelatine-Lösung bei 60° C in einem Ultraschallbad gelöst und darauf circa 1 Stunde auf einer Temperatur von 80° C bis 85° C gehalten. Es werden 6 Gew% Gentamicinsulfat (Produktnummer: 345814, Merck, Germany) unter Rühren in der so hergestellten heißen Gelatinelösung gelöst.

Die auf 80° C bis 85° C temperierte Gelatine-Lösung wird mittels einer Spritzenpumpe als Faserrohmaterial in das Behältnis einer Vorrichtung zum Rotationsspinnen gemäß der DE 10 2005 048 939 A1 geführt. Über eine zweite Spritzenpumpe wird gleichzeitig Polyethylenglycoldiglycidylether (Produktnummer: 475696, Sigma-Aldrich, Germany) in das Behältnis geführt.

Das Behältnis hat eine Temperatur von circa 120° C und rotiert mit einer Drehzahl von 4500 Umdrehungen/min. Im Behältnis befinden sich Ausnehmungen, die als Löcher mit einem Durchmesser von 0,3 mm ausgestaltet sind. Durch die Zentrifugalkraft wird das Faserrohmaterial durch die Ausnehmungen gepresst und zu Fasern 1 gesponnen, die durch eine Absaugeinrichtung verstreckt werden. Die Absaugeinrichtung befindet sich unterhalb des Behältnisses.

Die Faserdurchmesser wurden mit Hilfe eines Zeiss Stemi 2000-C Mikroskops bestimmt. Hierfür wurde der Mittelwert von 10 Einzelmessungen bestimmt.

Zur Bestimmung der Flächengewichte dienten 10x10 cm$^2$ Vliesproben. Die Gewichte wurden mit Hilfe eine Mikroanalysenwage der Firma Satorius (Modell Acculab VIC-123) bestimmt.

Die Dicke der Vliesproben wurde mittels eines Dickenmessgeräts der Firma Schräder (Modell Dickenmessgerät RAINBOW) gemessen. Dabei darf zur Bestimmung der Dicke kein Druck auf das Vlies ausgeübt werden, damit es zu keiner ungewollten Komprimierung des Vlieses kommt und dadurch zu einer Verkleinerung des Volumens.

Die Porosität ε der Proben wurde nach folgender Formel berechnet:

$$\varepsilon = 1 - \frac{\rho_{Vlies}}{\rho_{Bulk}}$$

$$= 1 - \frac{\frac{m_{Vlies}}{V_{Vlies}}}{\rho_a \cdot \frac{m_a}{(m_a + m_b + m_c)} + \rho_b \cdot \frac{m_b}{(m_a + m_b + m_c)} + \rho_c \cdot \frac{m_c}{(m_a + m_b + m_c)}}$$

Darin ist $\rho_{Vlies}$ die Dichte des nicht komprimierten biodegradierbaren Vlieses, $\rho_{Bulk}$ die Dichte der Fasern 1 des biodegradierbaren Vlieses, $m_{Vlies}$ die Masse des biodegradierbaren-Vlieses, $V_{Vlies}$ das Volumen des biodegradierbaren Vlieses, $\rho_a$ die Dichte des faserbildenden Polymers, $\rho_b$ die Dichte des Aktivators der sekundären Hämostase, $\rho_c$ die Dichte des Inhibitors der Fibrinolyse, $m_a$ die Masse des faserbildenden Polymers im Vlies, $m_b$ die Masse des Aktivators der sekundären Hämostase im Vlies und $m_c$ die Masse des Inhibitors der Fibrinolyse im Vlies. Wenn noch weitere Komponenten, wie beispielsweise eine zusätzliche Puffersubstanz oder Antibiotika im biodegradierbaren Vlies enthalten sind und die Porosität des Vlieses bestimmt werden soll, müssen noch weitere Parameter nach obigem Muster berücksichtigt werden, das heißt die Massen ($m_d$, $m_e$, ...) und Dichten ($\rho_d$, $\rho_e$, ...) der zusätzlichen Komponenten. Der mittlere Porenradius wurde nach S. J. Eichhorn, W. W. Sampson, Statistical geometry of pores and statistics of porous nanofibrous assemblies, J. R. Soc. Interface, 2005; 2; 309-318 berechnet. Hierzu diente die Formel 6.1 auf Seite 315.

Die Faseroberfläche $O_{Faser}$ wurde nach folgender Formel berechnet:

$$O_{Faser} = \pi \cdot \emptyset_{Faser} \cdot \frac{1 - \varepsilon \cdot V_{Vlies}}{\pi \left(\frac{\emptyset_{Faser}}{2}\right)^2},$$

wobei $\emptyset_{Faser}$ der durchschnittliche Durchmesser der Fasern 1 ist. Der Kontaktwinkel wurde mit einem Goniometer G40 (der Firma Krüss) bestimmt. Hierbei wurden Wassertropfen auf die Vliesoberfläche gesetzt und der Benetzungswinkel nach 10 s gemessen.

Durchschnittlicher Faserdurchmesser $\emptyset_{Faser}$: 14 ± 5 μm
Flächengewicht: 200 g/m$^2$
Dicke der Proben: 2 mm
Porosität: 0,919
Mittlerer Porenradius: 0,1094 mm
Gesamtfaseroberfläche: 462,072 mm$^2$
Kontaktwinkel: < 30°

AUSFÜHRUNGSBEISPIEL 2:

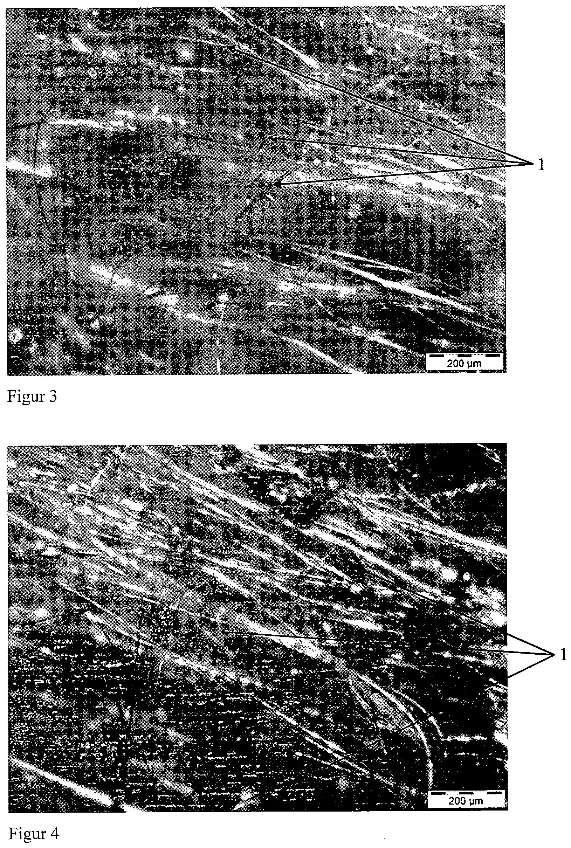

Ein Gelatinevliesstoff mit antimikrobieller Beschichtung gemäß Fig. 3 wird durch ein Rotationsspinnverfahren wie folgt hergestellt:

Zunächst wird eine 24%ige Gelatine-Lösung hergestellt. Zur Verwendung kommt eine Gelatine des Typs A PIGSKIN der Firma GELITA AG. Die Gelatine wird in Wasser eingerührt. Der pH-Wert wird mit NaOH (Produktnummer: 3306576, Sigma-Aldrich, Germany) auf 7,4 eingestellt. Der Gelatine-Lösung werden 1 Gew% Calciumchlorid (Produktnummer: 102382, Merck, Germany), 1 Gew% Calciumcarbonat (Produktnummer: C4830, Sigma-Aldrich, Germany), 1 Gew% Glycerin (Produktnummer: 01873, Sigma-Aldrich, Germany), 0,5 Gew% trans-4-Aminomethylcyclohexylcarbonsäure (Produktnummer: 857653, Sigma-Aldrich, Germany) und 10 mg Bromkresolpur- pur (Produktnummer: 114375, Sigma-Aldrich, Germany) zugegeben.

Darauf verbleibt die Gelatine-Lösung etwa eine Stunde in Ruhe, um aufzuquellen. Anschließend wird die Gelatine-Lösung bei 60° C in einem Ultraschallbad gelöst und darauf ca. 1 Stunde auf einer Temperatur von 80° C bis 85° C gehalten.

Die auf 80° C bis 85° C temperierte Gelatine-Lösung wird mittels einer Spritzenpumpe als Faserrohmaterial in das Behältnis einer Vorrichtung zum Rotationsspinnen gemäß der DE 10 2005 048 939 A1 geführt. Über eine zweite Spritzenpumpe wird gleichzeitig Polyethylenglycoldiglycidylether (Produktnummer: 475696, Sigma-Aldrich, Germany) in das Behältnis geführt.

Das Behältnis hat eine Temperatur von circa 120° C und rotiert mit einer Drehzahl von 4500 U/min. Im Behältnis befinden sich Ausnehmungen, die als Löcher mit einem Durchmesser von 0,3 mm ausgestaltet sind. Durch die Zentrifugalkraft wird das Faserrohmaterial durch die Ausnehmungen gepresst und zu Fasern 1 gesponnen, die durch eine Absaugeinrichtung verstreckt werden. Die Absaugeinrichtung befindet sich unterhalb des Behältnisses.

Das so erhaltene Vlies wird mit einer Gentamicinpalmitat-Lösung (Heraeus Medical, Germany) (5 g in 100 ml Methanol gelöst) besprüht und im Vakuum getrocknet.

AUSFÜHRUNGSBEISPIEL 3:

Ein Gelatinevliesstoff mit antimikrobiellem Stoff und antiseptischer Beschichtung gemäß Figur 4 wird durch ein Rotationsspinnverfahren wie folgt hergestellt:

Zunächst wird eine 24%ige Gelatine-Lösung hergestellt. Vorliegend wurde eine Gelatine des Typs A PIGSKIN der Firma GELITA AG eingesetzt, wobei andere Gelatinetypen ebenfalls verwendet werden können. Die Gelatine wird in Wasser eingerührt. Der pH-Wert wird mit NaOH (Produktnummer: 3306576, Sigma-Aldrich, Germany) auf 7,4 eingestellt. Der Gelatine-Lösung werden 1 Gew% Calciumchlorid (Produktnummer: 102382, Merck, Germany), 5 Gew% Calciumcarbonat (Produktnummer: C4830, Sigma-Aldrich, Germany), 1 Gew% Glycerin (Produktnummer: 01873, Sigma-Aldrich, Germany), 0,5 Gew% 6-Aminohexansäure (Produktnummer: 800145, Merck, Germany) und 10 mg Bromthymolblau (Produktnummer: 114413, Sigma-Aldrich, Germany) zugegeben. Darauf verbleibt die Gelatine-Lösung etwa eine Stunde in Ruhe, um aufzuquellen. Anschließend wird die Gelatine-Lösung bei 60° C in einem Ultraschallbad gelöst und darauf ca. 1 Stunde auf einer Temperatur von 80° C bis 85° C gehalten. Es werden 6 Gew% Gentamicinsulfat (Produktnummer: 345814, Merck, Germany) unter Rühren in der so hergestellten heißen Gelatinelösung gelöst.

Die auf 80° C bis 85° C temperierte Gelatine-Lösung wird mittels einer Spritzenpumpe als Faserrohmaterial in das Behältnis einer Vorrichtung zum Rotationsspinnen gemäß der DE 10 2005 048 939 A1 geführt.

Das Behältnis hat eine Temperatur von circa 120° C und rotiert mit einer Drehzahl von 4500 Umdrehungen/min. Im Behältnis befinden sich Ausnehmungen, die als Löcher mit einem Durchmesser von 0,3 mm ausgestaltet sind. Durch die Zentrifugalkraft wird das Faserrohmaterial durch die Ausnehmungen gepresst und zu Fasern 1 gesponnen, die durch eine Absaugeinrichtung verstreckt werden. Die Absaugeinrichtung befindet sich unterhalb des Behältnisses.

Der so erhaltene Vliesstoff wird in einem Exsikkator für 12 Stunden bei Raumtemperatur in Gegenwart einer 36%igen Formaldehydlösung (Produktnummer: F8775, Sigma-Aldrich, Germany) gelagert und anschließend zur vollständigen Entfernung des überschüssigen Formaldehyds für weitere 72 Stunden evakuiert. Anschließend wird das Vlies mit einer Polyhexanid-Lösung (Hangzhou Dayangchem Co., Ltd., China) (5 g Polyhexanid in 100 ml einer Ethanol/Wasser-Mischung (80/20; V/V) besprüht und im Vakuum getrocknet.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen. Abschließend sei hervorgehoben, dass die zuvor rein willkürlich ausgewählten Ausführungsbeispiele lediglich zur Erörterung der erfindungsgemäßen Lehre dienen, diese jedoch nicht auf diese Ausführungsbeispiele einschränken.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

**BEZUGSZEICHENLISTE**

1    Faser / Filament

**Patentansprüche**

1.   Biodegradierbares Vlies enthaltend

    (i) wenigstens ein Polymer zum Induzieren der primären Hämostase,
    (ii) wenigstens einen nicht-proteinogenen, niedermolekularen, in Wasser löslichen Aktivator der sekundären Hämostase und
    (iii) wenigstens einen nicht-proteinogenen, niedermolekularen, in Wasser löslichen Inhibitor der Fibrinolyse.

2.   Biodegradierbares Vlies nach Anspruch 1, **dadurch gekennzeichnet, dass** das Vlies wenigstens einen antiinfektiven Wirkstoff aufweist.

3.   Biodegradierbares Vlies nach Anspruch 1 oder 2, **dadurch gekennzeichnet, dass** das Vlies Fasern (1) aufweist, wobei die Fasern (1) des Vlieses (i) das wenigstens eine Polymer zum Induzieren der primären Hämostase, (ii) den nicht-proteinogenen, niedermolekularen, in Wasser löslichen Aktivator der sekundären Hämostase, (iii) den nicht-proteinogenen, niedermolekularen, in Wasser löslichen Inhibitor der Fibrinolyse und/oder gegebenenfalls den wenigstens einen antiinfektiven Wirkstoff umfassen.

4.   Biodegradierbares Vlies nach einem der Ansprüche 1 bis 3, **dadurch gekennzeichnet, dass** das Vlies trocken ist und die durchschnittliche Maschenweite zwischen den Fasern (1) des trockenen Vlieses wenigstens 50 $\mu$m beträgt und vorzugsweise im Bereich von 50 $\mu$m bis 500 $\mu$m und mehr bevorzugt im Bereich von 100 $\mu$m bis 200 $\mu$m liegt.

5.   Biodegradierbares Vlies nach einem der Ansprüche 1 bis 4, **dadurch gekennzeichnet, dass** das Polymer, das die primäre Hämostase induziert, aus der Gruppe ausgewählt ist, die aus Kollagen, Gelatine, Carboxymethylcellulose, Oxycellulose, Carboxymethyldextran und Mischungen davon besteht.

6.   Biodegradierbares Vlies nach einem der Ansprüche 1 bis 5, **dadurch gekennzeichnet, dass** der Aktivator der sekundären Hämostase wenigstens ein Calciumsalz ist, vorzugsweise wenigstens ein Calciumsalz mit einer Löslichkeit von mehr als 2 g/Liter in Wasser bei einer Temperatur von 25°C, wobei das wenigstens eine Calciumsalz besonders bevorzugt aus der Gruppe ausgewählt ist, die aus Calciumchlorid, Calciumacetat, Calciumsulfat-Dihydrat, Calciumlactat und Mischungen davon besteht.

7.   Biodegradierbares Vlies nach einem der vorangehenden Ansprüche, **dadurch gekennzeichnet, dass** der nicht-proteinogene, niedermolekulare, in Wasser lösliche Inhibitor der Fibrinolyse aus der Gruppe ausgewählt ist, die aus amphoteren Aminocarbonsäuren besteht, und mehr bevorzugt aus der Gruppe ausgewählt ist, die aus 6-Aminohexansäure, 4-Aminomethylbenzoesäure und trans-4-Aminomethylcyclohexylcarbonsäure besteht.

8. Biodegradierbares Vlies nach einem der vorangehenden Ansprüche, **dadurch gekennzeichnet, dass** der Anteil des Inhibitors der Fibrinolyse im Bereich von 0,1 bis 20 Gewichtsprozent, vorzugsweise im Bereich von 0,5 bis 15 Gewichtsprozent und mehr bevorzugt im Bereich von 1 bis 10 Gewichtsprozent, bezogen auf das Gewicht des Vlieses liegt.

9. Biodegradierbares Vlies nach einem der vorangehenden Ansprüche, **dadurch gekennzeichnet, dass** das Vlies wenigstens eine in Wasser gering lösliche Puffersubstanz umfasst, die eine Löslichkeit von weniger als 10 g und vorzugsweise von weniger als 5 g, pro Liter Wasser bei einer Temperatur von 25°C aufweist, wobei die Puffersubstanz besonders bevorzugt aus der Gruppe ausgewählt ist, die aus Calciumcarbonat, Magnesiumcarbonat, basischem Magnesiumcarbonat und Mischungen davon besteht.

10. Biodegradierbares Vlies nach einem der vorangehenden Ansprüche, **dadurch gekennzeichnet, dass** das Vlies einen pH-Indikator umfasst, der vorzugsweise einen Umschlagpunkt bei einem pH-Wert von weniger als pH 7,4 hat, wobei der pH-Indikator besonders bevorzugt aus der Gruppe ausgewählt ist, die aus Bromkresolpurpur und Bromthymolblau besteht.

11. Biodegradierbares Vlies nach einem der vorangehenden Ansprüche, **dadurch gekennzeichnet, dass** wenigstens ein antiinfektiver Wirkstoff vorzugsweise wenigstens ein Antibiotikum, auf der Vliesoberfläche angeordnet ist, wobei der wenigstens eine antiinfektive Wirkstoff vorzugsweise in Wasser löslich ist.

12. Biodegradierbares Vlies nach einem der vorangehenden Ansprüche, **dadurch gekennzeichnet, dass** das Vlies Fasern (1) aufweist und die Fasern (1) des Vlieses einen mittleren Faserdurchmesser im Bereich von 0,5 $\mu$m bis 500 $\mu$m, vorzugsweise im Bereich von 2 $\mu$m bis 300 $\mu$m und mehr bevorzugt im Bereich von 5 $\mu$m bis 200 $\mu$m haben.

13. Biodegradierbares Vlies nach einem der vorangehenden Ansprüche, **dadurch gekennzeichnet, dass** die Menge des nicht-proteinogenen, niedermolekularen, in Wasser löslichen Inhibitors der Fibrinolyse derart gewählt ist, dass dieser eine den pH-Wert stabilisierende Pufferwirkung im Bereich der Oberfläche des Vlieses hat, wobei vorzugsweise der Inhibitor den pH-Wert im Bereich zwischen 6 und 8 puffert.

14. Verfahren zur Herstellung eines biodegradierbaren Vlieses nach einem der vorangehenden Ansprüche, **dadurch gekennzeichnet, dass**

(i) ein fluidisiertes Faserrohmaterial gegebenenfalls mit Additiven in ein Behältnis gegeben wird,
(ii) das Behältnis in Rotation versetzt wird,
(iii) das fluidisierte Faserrohmaterial durch Zentrifugalkräfte aus dem Behältnis ausgetragen wird, wodurch Fasern (1) oder Filamente (1) gebildet werden, und
(iv) aus den Fasern (1) oder Filamenten (i) ein biodegradierbares Vlies gebildet wird.

15. Verfahren nach Anspruch 14, **dadurch gekennzeichnet, dass** die erzeugten Fasern (1) oder Filamente (1) aus dem rotierenden Behältnis als ein Flächenmaterial aufgefangen werden, in dem sich in einer Vielzahl von Bereichen des Flächenmaterials Verbindungsstellen zwischen zwei oder mehreren Fasern (1) bilden.

16. Verfahren nach Anspruch 14 oder 15, **dadurch gekennzeichnet, dass** das Vlies, insbesondere das Flächenmaterial, in wenigstens einem Nachbehandlungsschritt mit wenigstens einem flüssigen Medium getränkt und/oder beschichtet wird, wobei insbesondere als flüssiges Medium biologisch abbaubare Polymermaterialien und/oder wachsartige Materialien verwendet werden.

**Claims**

1. Biodegradable fleece, containing

(i) at least one polymer for induction of primary haemostasis,
(ii) at least one non-proteinogenic, low molecular, water-soluble activator of secondary haemostasis, and
(iii) at least one non-proteinogenic, low molecular, water-soluble inhibitor of fibrinolysis.

**2.** Biodegradable fleece according to claim 1, **characterised in that** the fleece contains at least one anti-infective agent.

**3.** Biodegradable fleece according to claim 1 or 2, **characterised in that** the fleece comprises fibres (1), whereby the fibres (1) of the fleece comprise (i) the at least one polymer for induction of primary haemostasis, (ii) the non-proteinogenic, low molecular, water-soluble activator of secondary haemostasis, and (iii) the non-proteinogenic, low molecular, water-soluble inhibitor of fibrinolysis and/or, if applicable, the at least one anti-infective agent.

**4.** Biodegradable fleece according to any one of the claims 1 to 3, **characterised in that** the fleece is dry and the average mesh width between the fibres (1) of the dry fleece is at least 50 $\mu$m and preferably is in the range of 50 $\mu$m to 500 $\mu$m, and more preferably is in the range of 100 $\mu$m to 200 $\mu$m.

**5.** Biodegradable fleece according to any one of the claims 1 to 4, **characterised in that** the polymer inducing the primary haemostasis is selected from the group consisting of collagen, gelatin, carboxymethylcellulose, oxycellulose, carboxymethyldextran, and mixtures thereof.

**6.** Biodegradable fleece according to any one of the claims 1 to 5, **characterised in that** the activator of secondary haemostasis is at least one calcium salt, preferably at least one calcium salt with a solubility of more than 2 g/litre in water at a temperature of 25°C, whereby the at least one calcium salt particularly preferably is selected from the group consisting of calcium chloride, calcium acetate, calcium sulfate dihydrate, calcium lactate, and mixtures thereof.

**7.** Biodegradable fleece according to any one of the preceding claims, **characterised in that** the non-proteinogenic, low molecular, water-soluble inhibitor of fibrinolysis is selected from the group consisting of amphoteric aminocarboxylic acids, and more preferably is selected from the group consisting of 6-aminohexanoic acid, 4-aminomethylbenzoic acid, and trans-4-aminomethylcyclohexylcarboxylic acid.

**8.** Biodegradable fleece according to any one of the preceding claims, **characterised in that** the fraction of the inhibitor of fibrinolysis is in the range of 0.1 to 20% by weight, preferably in the range of 0.5 to 15% by weight, and more preferably in the range of 1 to 10% by weight, relative to the weight of the fleece.

**9.** Biodegradable fleece according to any one of the preceding claims, **characterised in that** the fleece comprises at least one poorly water-soluble buffer substance that has a solubility of less than 10 g, and preferably of less than 5 g, per litre of water at a temperature of 25°C, whereby the buffer substance particularly preferably is selected from the group consisting of calcium carbonate, magnesium carbonate, basic magnesium carbonate, and mixtures thereof.

**10.** Biodegradable fleece according to any one of the preceding claims, **characterised in that** the fleece comprises a pH indicator, which preferably has a point of change at a pH value of less than pH 7.4, whereby the pH indicator particularly preferably is selected from the group consisting of bromocresol purple and bromothymol blue.

**11.** Biodegradable fleece according to any one of the preceding claims, **characterised in that** at least one anti-infective agent, preferably at least one antibiotic, is arranged on the fleece surface, whereby the at least one anti-infective agent preferably is soluble in water.

**12.** Biodegradable fleece according to any one of the preceding claims, **characterised in that** the fleece comprises fibres (1) and the fibres (1) of the fleece have a mean fibre diameter in the range of 0.5 $\mu$m to 500 $\mu$m, preferably in the range of 2 $\mu$m to 300 $\mu$m, and more preferably in the range of 5 $\mu$m to 200 $\mu$m.

**13.** Biodegradable fleece according to any one of the preceding claims, **characterised in that** the amount of the non-proteinogenic, low molecular, water-soluble inhibitor of fibrinolysis is selected appropriately such that the same has a pH value-stabilising buffer effect in the region of the surface of the fleece, whereby the inhibitor preferably buffers the pH value in the range between 6 and 8.

**14.** Method for producing a biodegradable fleece according to any one of the preceding claims, **characterised in that**

(i) a fluidised fibre raw material, possibly with additives, is placed in a container;
(ii) the container is made to rotate;
(iii) the fluidised fibre raw material is driven from the container by centrifugal forces, by means of which fibres (1) or filaments (1) are formed; and
(iv) a biodegradable fleece is formed from the fibres (1) or filaments [(1)$_{[VR1]}$.

**15.** Method according to claim 14, **characterised in that** the fibres (1) or filaments (1) produced in the method are caught, from the rotating container, in the form of a two-dimensional material, in which connecting sites between two or more fibres (1) form in a multitude of regions of the two-dimensional material.

**16.** Method according to claim 14 or 15, **characterised in that** the fleece, in particular the two-dimensional material, is soaked in and/or coated with at least one liquid medium in at least one follow-up treatment step, whereby, in particular, biologically degradable polymer materials and/or wax-like materials are used as liquid medium.

**Revendications**

**1.** Non-tissé biodégradable contenant

(i) au moins un polymère pour l'induction de l'hémostase primaire,
(ii) au moins un activateur de l'hémostase secondaire non protéinogène, à faible poids moléculaire, soluble dans l'eau, et
(iii) au moins un inhibiteur de la fibrinolyse non protéinogène, à faible poids moléculaire, soluble dans l'eau.

**2.** Non-tissé biodégradable selon la revendication 1, **caractérisé en ce que** le non-tissé présente au moins un ingrédient actif anti-infectieux.

**3.** Non-tissé biodégradable selon la revendication 1 ou 2, **caractérisé en ce que** le non-tissé présente des fibres (1), dans lequel les fibres (1) du non-tissé comprennent (i) l'au moins un polymère pour l'induction de l'hémostase primaire, (ii) l'activateur de l'hémostase secondaire non protéinogène, à faible poids moléculaire, soluble dans l'eau, (iii) l'inhibiteur de la fibrinolyse non protéinogène, à faible poids moléculaire, soluble dans l'eau et/ou éventuellement l'au moins un ingrédient actif anti-infectieux.

**4.** Non-tissé biodégradable selon une des revendications 1 à 3, **caractérisé en ce que** le non-tissé est sec et la largeur de mailles moyenne entre les fibres (1) du non-tissé sec est d'au moins 50 μm et se situe de préférence dans la région de 50 μm à 500 μm et de manière davantage préférée dans la région de 100 μm à 200 μm.

**5.** Non-tissé biodégradable selon une des revendications 1 à 4, **caractérisé en ce que** le polymère qui induit l'hémostase primaire est sélectionné parmi le groupe qui se compose du collagène, de la gélatine, de la carboxyméthylcellulose, de l'oxycellulose, de la carboxyméthyldextrane et de mélanges de ceux-ci.

**6.** Non-tissé biodégradable selon une des revendications 1 à 5, **caractérisé en ce que** l'activateur de l'hémostase secondaire est au moins un sel de calcium, de préférence au moins un sel de calcium avec une solubilité de plus de 2 g/litre dans l'eau à une température de 25°C, dans lequel l'au moins un sel de calcium est de manière particulièrement préférée sélectionné parmi le groupe qui se compose du chlorure de calcium, de l'acétate de calcium, du dihydrate de sulfate de calcium, du lactate de calcium et de mélanges de ceux-ci.

**7.** Non-tissé biodégradable selon une des revendications précédentes, **caractérisé en ce que** l'inhibiteur de la fibrinolyse non protéinogène, à faible poids moléculaire, soluble dans l'eau est sélectionné parmi le groupe qui se compose d'acides aminocarboxyliques amphotères, et est de manière davantage préférée sélectionné parmi le groupe qui se compose de l'acide 6-aminohexanoïque, de l'acide 4-aminométhylbenzoïque et de l'acide trans-4-aminométhylcyclohexylcarboxylique.

**8.** Non-tissé biodégradable selon une des revendications précédentes, **caractérisé en ce que** la proportion de l'inhibiteur de la fibrinolyse se situe dans la région de 0,1 à 20 pourcent en poids, de préférence dans la région de 0,5 à 15 pourcent en poids et de manière davantage préférée dans la région de 1 à 10 pourcent en poids, par rapport au poids du non-tissé.

**9.** Non-tissé biodégradable selon une des revendications précédentes, **caractérisé en ce que** le non-tissé comprend au moins une substance tampon faiblement soluble dans l'eau qui présente une solubilité de moins de 10 g et de préférence de moins de 5 g par litre d'eau à une température de 25°C, dans lequel la substance tampon est de manière particulièrement préférée sélectionnée parmi le groupe qui se compose du carbonate de calcium, du carbonate de magnésium, du carbonate de magnésium basique et de mélanges de ceux-ci.

**10.** Non-tissé biodégradable selon une des revendications précédentes, **caractérisé en ce que** le non-tissé comprend un indicateur de pH qui a de préférence un point final de la réaction à une valeur de pH de moins de pH 7,4, dans lequel l'indicateur de pH est de manière particulièrement préférée sélectionné parmi le groupe qui se compose du pourpre de bromocrésol et du bleu de bromothymol.

**11.** Non-tissé biodégradable selon une des revendications précédentes, **caractérisé en ce qu'**au moins un ingrédient actif anti-infectieux, de préférence au moins un antibiotique, est disposé sur la surface du non-tissé, dans lequel l'au moins un ingrédient actif anti-infectieux est de préférence soluble dans l'eau.

**12.** Non-tissé biodégradable selon une des revendications précédentes, **caractérisé en ce que** le non-tissé présente des fibres (1) et les fibres (1) du non-tissé ont un diamètre de fibre moyen dans la région de 0,5 $\mu$m à 500 $\mu$m, de préférence dans la région de 2 $\mu$m à 300 $\mu$m et de manière davantage préférée dans la région de 5 $\mu$m à 200 $\mu$m.

**13.** Non-tissé biodégradable selon une des revendications précédentes, **caractérisé en ce que** la quantité de l'inhibiteur de la fibrinolyse non protéinogène, à faible poids moléculaire, soluble dans l'eau est choisie de telle sorte que celle-ci a une action tampon de stabilisation de valeur de pH dans la région de la surface du non-tissé, dans lequel l'inhibiteur tamponne de préférence la valeur de pH dans la région entre 6 et 8.

**14.** Procédé de fabrication d'un non-tissé biodégradable selon une des revendications précédentes, **caractérisé en ce que**

(i) un matériau brut fibreux fluidisé éventuellement avec des additifs est versé dans un récipient,
(ii) le récipient est mis en rotation,
(iii) le matériau brut fibreux fluidisé est évacué du récipient par des forces centrifuges, moyennant quoi des fibres (1) ou filaments (1) sont formé(e)s, et
(iv) un non-tissé biodégradable est formé à partir des fibres (1) ou filaments (1).

**15.** Procédé selon la revendication 14, **caractérisé en ce que** les fibres (1) ou filaments (1) généré(e)s sont recueilli(e)s du récipient en rotation comme un matériau de surface dans lequel des points de connexion entre deux ou plusieurs fibres (1) se forment dans une pluralité de régions du matériau de surface.

**16.** Procédé selon la revendication 14 ou 15, **caractérisé en ce que** le non-tissé, notamment le matériau de surface, est imprégné et/ou enduit d'au moins un milieu fluide dans au moins une étape de traitement postérieur, dans lequel des matériaux polymères dégradables biologiquement et/ou matériaux cireux sont notamment utilisés comme milieu fluide.

Figur 1

Figur 2

Figur 3

Figur 4

**EP 2 812 037 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008076722 A1
- DE 102007011606 A1
- WO 2011116848 A
- DE 102007044648 A1
- DE 102005048939 A1

**In der Beschreibung aufgeführte Nicht-Patentliteratur**

- **A. K. LYNN ; I. V. YANNAS ; W. BONFIELD.** Antigenicity and immunogenicity of collagen. *J Biomed Mater Res B Appl Biomater,* 2004, vol. 71 (2), 343-354
- **Y. TOMIZAWA.** Clinical benefits and risk analysis of topical hemostats: a review. *J. Artif. Organs.,* 2005, vol. 8 (3), 137-142
- **S. SRINATH.** Topical hemostatic agents in surgery: A surgeon's perspective. *Aorn Journal.,* 2008, vol. 88 (3), 2-11
- **H. E. ACHNECK ; B. SILESHI ; R. M. JAMIOLKOWSKI ; D. A. ALBALA ; M. L. SHAPIRO ; J. H. LAWSON.** A comprehensive review of topical hemostatic agents: Efficacy and recommendations for use. *Anals of Surgery,* 2010, vol. 251 (2), 217-228
- **H. SEYEDEJAD ; M. IMANI ; T. JAMIESON ; A. M. SEIFALIAN.** Topical haemostatic agents. *British Journal of Surgery,* 2008, vol. 95, 1197-1225
- **Y. WAI ; V. TSUI ; Z. PENG ; R. RICHARDSON ; D. OREOPOULOS ; S. M. TARLO.** Anaphylaxis from topical bovine thrombin during haemodialysis and evaluation of sensitization among dialysis poulation. *Clin Exp Allergy,* 2003, vol. 33, 1730-1734
- **M. POPE ; K. W. JOHNSTON.** Anaphylaxis after thrombin injection of a femoral pseudoaneurysm: recommendations for prevention. *J Vase Surg,* 2000, vol. 32, 190-191
- **K. TADOKORO ; T.OHTOSHI ; S. TAKAFUIJI ; K. NAKAJIMA ; S. SUZUKI ; K. YAMAMOTO et al.** Topical thrombin-induced IgE-mediated anaphylaxis: RAST analysis and skin-test studies. *J Allergy Clin Immunol,* 1991, vol. 88, 620-629
- **K. AKTORIES ; U. FÖRSTERMANN ; W. FORTH.** Allgemeine und spezielle Pharmakologie und Toxikologie. Elsevier, Urban&Fischer Verlag, 2006, 547
- **R. N. KADDOUM ; E. J. CHIDIAC ; M. M. ZESTOS ; S. D. RAJAN ; A. BARAKA.** An anaphylactic reaction after primary exposure to an aprotinin test dose in a child with a severe milk allergy. *J. Cardiothorac. Vasc. Anesth.,* 2007, vol. 21, 243-244
- **W. DIETRICH ; A. EBELL ; R. BUSLEY ; A. BOULESTEIX.** Aprotinin and anaphylaxis: analysis of 12403 exposures to aprotinin in cardiac surgery. *Ann. Thorac. Surg.,* 2007, vol. 84, 1144-1150
- **J. DISSEMOND.** Die Bedeutung des pH-Wertes für die Wundheilung. *HARTMANN wundForum,* 2006, vol. 1, 15-19
- **W. F. KONERTZ ; M. KOSTELKA ; F. W. MOHR et al.** Reducing the incidence and severity of pericardial adhesions with a sprayable polymeric matrix. *Ann. Thorac. Surg.,* 2003, vol. 76, 1270-1274
- Produktnummer. Sigma-Aldrich, vol. 475696
- **S. J. EICHHORN ; W. W. SAMPSON.** Statistical geometry of pores and statistics of porous nanofibrous assemblies. *J. R. Soc. Interface,* 2005, vol. 2, 309-318

17